(19) Europäisches Patentamt
European Patent Office
Office européen des brevets

(11) **EP 1 315 770 B1**

(12) **EUROPÄISCHE PATENTSCHRIFT**

(45) Veröffentlichungstag und Bekanntmachung des Hinweises auf die Patenterteilung:
**23.08.2006 Patentblatt 2006/34**

(21) Anmeldenummer: **01947342.0**

(22) Anmeldetag: **05.06.2001**

(51) Int Cl.:
**C08J 3/24** *(2006.01)*    **C08L 101/14** *(2006.01)*
**A61L 15/60** *(2006.01)*

(86) Internationale Anmeldenummer:
**PCT/EP2001/006375**

(87) Internationale Veröffentlichungsnummer:
**WO 2002/022717 (21.03.2002 Gazette 2002/12)**

(54) **VERWENDUNG VON PULVERFÖRMIGEN, VERNETZTEN, WÄSSRIGE FLÜSSIGKEITEN ABSORBIERENDEN POLYMEREN IN HYGIENEARTIKEL**

USE OF PULVERULENT, CROSSLINKED POLYMERS, CAPABLE OF ABSORBING AQUEOUS LIQUIDS, IN HYGIENIC ARTICLES

UTILISATION DE POLYMERES RETICULES PULVERULENTS ABSORBANT DES LIQUIDES AQUEUX DANS LES ARTICLES D'HYGIENE

(84) Benannte Vertragsstaaten:
**AT BE CH CY DE DK ES FI FR GB GR IE IT LI LU MC NL PT SE TR**

(30) Priorität: **04.09.2000 DE 10043710**

(43) Veröffentlichungstag der Anmeldung:
**04.06.2003 Patentblatt 2003/23**

(73) Patentinhaber: **Stockhausen GmbH**
**47805 Krefeld (DE)**

(72) Erfinder:
• **MERTENS, Richard**
**47803 Krefeld (DE)**
• **HARREN, Jörg**
**47807 Krefeld (DE)**

(74) Vertreter: **Herzog, Martin et al**
**Kahlhöfer . Neumann . Herzog . Fiesser,**
**Karlstrasse 76**
**40210 Düsseldorf (DE)**

(56) Entgegenhaltungen:
**WO-A-00/53664        JP-A- 9 124 879**

**Beschreibung**

Die Erfindung betrifft die Verwendung pulverförmiger, an der Oberfläche nachvernetzter Wasser, wässrige oder seräse Flüssigkeiten sowie Blut absorbierende Polymere (Superabsorber), mit verbesserten Eigenschaften insbesondere mit einer verbesserten Retention und einem verbesserten Rückhaltevermögen von Flüssigkeiten unter Druck und einer verbesserten Fähigkeit Flüssigkeiten zu transportieren, als Absorptionsmitttel in Hygieneartikeln.

Superabsorber sind wasserunlösliche, vernetzte Polymere, die in der Lage sind, unter Quellung und Ausbildung von Hydrogelen große Mengen an wässrigen Flüssigkeiten und Körperflüssigkeiten, wie z. B. Urin oder Blut, aufzunehmen und unter einem bestimmten Druck zurückzuhalten. Durch diese charakteristischen Eigenschaften finden diese Polymere hauptsächlich Anwendung bei der Einarbeitung in Sanitärartikel, wie z. B. Babywindeln, Inkontinenzprodukten oder Damenbinden.

Bei den gegenwärtig kommerziell verfügbaren Superabsorbern handelt es sich im Wesentlichen um vernetzte Polyacrylsäuren oder vernetzte Stärke-Acrylsäure-Pfropfpolymerisate, bei denen die Carboxylgruppen teilweise mit Natronlauge oder Kalilauge neutralisiert sind.

Aus ästhetischen Gründen und aus Umweltaspekten besteht die zunehmende Tendenz, die Sanitärartikel wie Babywindeln, Inkontinenzprodukte und Damenbinden immer kleiner und dünner zu gestalten. Um ein gleichbleibendes Gesamtretentionsvermögen der Sanitärartikel zu gewährleisten, kann dieser Anforderung nur durch Reduktion des Anteils an großvolumigen Fluff entsprochen werden. Hierdurch fallen dem Superabsorber weitere Aufgaben hinsichtlich Transport und Verteilung von Flüssigkeit zu, die sich als Permeabilitätseigenschaften zusammenfassen lassen.

Unter Permeabilität versteht man bei Superabsorbermaterialien die Fähigkeit, im gequollenen Zustand zugegebene Flüssigkeiten zu transportieren und dreidimensional zu verteilen. Dieser Prozeß läuft im gequollenen Superabsorbergel über kapillaren Transport durch Zwischenräume zwischen den Gelpartikeln ab. Ein Flüssigkeitstransport durch gequollene Superabsorberpartikel selbst folgt den Gesetzen der Diffusion und ist ein sehr langsamer Prozeß, der in der Nutzungssituation des Sanitärartikels keine Rolle bei der Verteilung der Flüssigkeit spielt. Bei Superabsorbermaterialien, die einen kapillaren Transport aufgrund mangelnder Gelstabilität nicht bewerkstelligen können, wurde durch Einbetten dieser Materialien in eine Fasermatrix eine Separation der Partikel voneinander unter Vermeidung des Gel-Blocking-Phänomens sichergestellt. In Windelkonstruktionen neuer Generation befindet sich in der Absorberschicht nur wenig oder überhaupt kein Fasermaterial zur Unterstützung des Flüssigkeitstransports. Die hier verwendeten Superabsorber müssen demnach eine ausreichend hohe Stabilität im gequollenen Zustand besitzen, damit das gequollene Gel noch eine ausreichende Menge an kapillaren Räumen besitzt, durch die Flüssigkeit transportiert werden kann.

Um Superabsorbermaterialien mit hoher Gelstärke zu erhalten, kann einerseits der Grad der Vernetzung des Polymers angehoben werden, was zwangsläufig eine Verminderung der Quellfähigkeit und des Retentionsvermögens zur Folge hat. Eine optimierte Kombination von verschiedenen Vernetzern und Comonomeren, wie in Patentschrift DE 196 46 484 beschrieben, vermag die Permeabilitätseigenschaften zwar zu verbessern, nicht aber auf ein Niveau, das beispielsweise den Einbau einer gegebenenfalls nur aus Superabsorbern bestehende Schicht in eine Windelkonstruktion erlaubt.

Weiterhin können Methoden zur oberflächlichen Nachvernetzung der Polymerpartikel zur Anwendung kommen. Bei der sog. Nachvernetzung werden die Carboxylgruppen der Polymermoleküle an der Oberfläche der Superabsorberpartikel mit verschiedenen Nachvernetzungsmitteln, die mit mindestens zwei der oberflächennahen Carboxylgruppen reagieren können, zur Reaktion gebracht. Neben der Erhöhung der Gelstärke wird insbesondere die Fähigkeit zur Flüssigkeitsaufnahme unter Druck stark verbessert, da das bekannte Phänomen des Gel-Blocking unterdrückt wird, bei dem angequollene Polymerteilchen verkleben und dadurch eine weitere Flüssigkeitsaufnahme verhindert wird.

Die Oberflächenbehandlung von flüssigkeitsabsorbierenden Harzen ist bereits bekannt. Zur Verbesserung der Dispergierbarkeit wird eine ionische Komplexierung der oberflächennahen Carboxylgruppen mit polyvalenten Metallkationen in der US 4,043,952 vorgeschlagen. Die Behandlung erfolgt mit Salzen mehrwertiger Metalle, die in organischen, ggf. Wasser enthaltende Solventien, (Alkohole und andere organische Solventien), dispergiert sind.

Eine Nachbehandlung von Superabsorberpolymeren mit reaktionsfähigen, oberflächenvernetzenden Verbindungen (Alkylencarbonate) zur Erhöhung der Flüssigkeitsaufnahmefähigkeit unter Druck wird in DE-A-40 20 780 beschrieben.

Eine Oberflächennachvernetzung von superabsorbierenden Polymeren mit polyfunktionellem Vernetzer wie polyvalenten Metallverbindungen in Gegenwart von inertem, anorganischem Pulver wie $SiO_2$ zur Verbesserung der Absorptionseigenschaften und zur Erzeugung eines nichtklebenden Gels der Polymerteilchen wird in DE-A-35 03 458 beschrieben.

Gemäß der Lehre von EP-A-0 574 260 erhält man superabsorbierende Polymere mit einem niedrigen Restmonomergehalt, der sich auch bei einer Oberflächenvernetzung nicht entscheidend verändert, wenn man bei der Polymerisation bestimmte Bedingungen einhält und die Nachvernetzung mit üblichen mehrfunktionellen Vernetzern, wie Polyolen, Alkylencarbonaten, polyvalenten Metallsalzen bei üblichen Bedingungen durchführt. Die nachvernetzten Polymeren zeigen eine gute Absorption ohne Druckanwendung.

Gemäß EP-A-0 889 063 können superabsorbierende Polymere, die bereits vorzugsweise oberflächenvernetzt sind, durch Nachbehandlung mit einer Verbindung des Titans oder Zirkons und eine diese Metallverbindungen chelatierende Verbindung gegen radikalischen Abbau durch Körperflüssigkeiten, insbesondere L-Askorbinsäure, ausgerüstet werden.

Die EP 0 233 067 beschreibt wasserabsorbierende, an der Oberfläche vernetzte Harze, die durch Reaktion von einem superabsorbierenden Polymerpulver mit 1 - 40 Gew.%, bezogen auf das Polymerpulver, einer Aluminiumverbindung erhalten werden. Als Behandlungslösung findet eine Mischung aus Wasser und Diolen Verwendung, die den Einsatz von niederen Alkoholen als Lösemittel überflüssig machen soll. Es werden bevorzugt 100 Gew.Teile Vernetzerlösung auf 100 bis 300 Gew.Teile Absorber aufgebracht. Die dem Reaktionsmedium Wasser zugefügten Diole (z. B. Polyethylenglycol 400 und 2000, 1,3-Butandiol oder 1,5-Pentandiol) dienen u. a. auch dazu, ein Verklumpen des Superabsorbers bei der Behandlung mit den hier verwendeten großen Mengen an wässriger Behandlungslösung zu verhindern. Das Lösemittel wird in einer anschließenden Trocknung bei 100°C entfernt. Die so behandelten Polymere weisen ein nicht ausreichendes Eigenschaftsniveau auf, wobei eine Verbesserung der Absorptionsfähigkeit unter Druck nicht erreicht wird. Außerdem ist eine Behandlung mit großen Mengen Behandlungslösung bei modernen, kontinuierlich arbeitenden Verfahren nicht ökonomisch durchführbar.

In der WO 96/05234 wird ein Verfahren zur Behandlung von superabsorbierenden Polymeren beschrieben, gemäß dem die Oberfläche der mindestens 10 Gew.% Wasser enthaltenden Absorberteilchen mit einer vernetzten Schicht erhalten durch eine Reaktion von einem reaktiven, hydrophilen Polymeren oder einer reaktiven metallorganischen Verbindung mit einem mindestens bifunktionellen Vernetzer bei Temperaturen unter 100°C ausgerüstet wurde. Metallsalze werden nicht aufgeführt. Die zum Einsatz kommenden Metallverbindungen müssen mit den funktionellen Gruppen des Vernetzers reagieren können. Als Metallverbindungen werden daher metallorganische Verbindungen empfohlen, die zu der Vernetzerverbindung im Gewichtsverhältnis 0,1 bis 30 vorliegen sollen. Die erhaltenen Polymerisate sollen ein ausgewogenes Verhältnis von Absorption, Gelfestigkeit und Permeabilität aufweisen, wobei die angegebenen Meßwerte unter weniger kritischen Bedingungen ermittelt werden. So werden beispielsweise die Absorption und die Permeabilität ohne jegliche Druckbelastung bestimmt. Nachteilig ist bei diesem bekannten Verfahren die Verwendung von Lösemitteln und toxisch bedenklichen Vernetzungsreagentien wie z.B. den als bevorzugt genannten Polyiminen, alkoxylierten Silan- bzw. Titan-Verbindungen und Epoxiden.

Durch eine entsprechende Behandlung von kommerziell erhältlichen Superabsorberpolymeren mit Aminopolymeren in organischen Lösungsmitteln wird gemäß der Lehre von WO 95/22356 und WO 97/12575 eine Verbesserung der Permeabilitäts- und Flüssigkeitstransporteigenschaften erreicht. Der gravierende Nachteil des hier beschriebenen Verfahrens liegt neben der Verwendung von toxikologisch bedenklichen Polyaminen und Polyiminen in dem Einsatz großer Mengen organischer Lösungsmittel, die für die Behandlung der Polymere notwendig sind. Der damit verbundene Sicherheitsaspekt und Kostenaufwand schließt eine großtechnische Produktion aus. Neben der toxikologischen Bedenklichkeit dieser Behandlungsmittel ist weiterhin zu berücksichtigen, daß sie unter den hohen Nachvernetzungstemperaturen auch zur Zersetzung neigen, was sich u.a. in einer Gelbfärbung der Absorberpartikel äußert.

Zur Herstellung von Wasser absorbierenden Polymeren mit besserer Abriebfestigkeit wird in der japanischen Offenlegungsschrift JP-A-09124879 die Oberflächennachvernetzung mit polyfunktionellen Vernetzer gelehrt, wobei der Wassergehalt der Polymerteilchen nach der Oberflächenvernetzung wieder auf 3 - 9 Gew.% eingestellt wid und diese Wassermenge anorganische Verbindungen wie Metallsalze enthalten kann.

Superabsorbierende Polymere, die gemäß WO 98/48857 in Teilchenform mit polyvalenten Metallsalzen durch trockenes Mischen in Kontakt gebracht und anschließend mit einer bestimmten Menge eines flüssigen Bindemittels, wie Wasser oder Polyole, versehen werden, sollen ein verbessertes Gelblocking bei der Absorption von wässrigen Flüssigkeiten aufweisen. Vor dieser Behandlung können die Polymerteilchen einer Oberflächennachvernetzung unterworfen werden.

Zur Minimierung der Agglomerationsneigung von superabsorbierenden, nachvernetzten Polymerteilchen durch elektrostatische Aufladung wird in WO 98/49221 empfohlen, die Polymerteilchen mit einer wäßrigen Additivlösungbis zu 10 Gew.% Wasser wieder zu befeuchten. Diese wäßrigen Lösungen können mono- oder polyvalente Ionen oder propoxylierte Polyole enthalten. Es ist auch möglich, die Polymerteilchen bereits vor der Oberflächennachbehandlung mit der wäßrigen Additivlösung in Kontakt zu bringen, wodurch eine gleichmäßigere Verteilung der Oberflächenbehandlungsmittel erreicht werden soll.

Einen Hinweis darauf, daß unter Beibehaltung einer hohen Retentionskapazität und Aufnahmefähigkeit von Flüssigkeit unter Druck bei der Nachvernetzungsstufe ebenfalls die Permeabilitätseigenschaften drastisch gesteigert werden können, ist aus dem vorstehend beschriebenen Stand der Technik nicht zu erkennen.

Aufgabe der vorliegenden Erfindung war es daher, superabsorbierende Polymere zu verwenden, die eine verbesserte Eigenschaftkombination aufweisen, insbesondere nicht nur eine hohe Aufnahmekapazität unter Druck, sondern auch die üblicherweise gegenläufigen Eigenschaften eines hohen Retentionsvermögens und einer guten Permeabilität in sich vereinigen, d. h. ein Niveau der Eigenschaftskombination aufweisen, bei dem neben einem Retentionswert von $\geq 25$ g/g mindestens ein SFC-Wert von mindestens $45 \cdot 10^{-7}$, vorzugsweise von mindestens $50 \cdot 10^{-7} \text{cm}^3$ s/g vorliegt. Insbesondere lag die Aufgabe darin, superabsorbierende Polymere zu verwenden in sehr dünnen Windelkonstruktionen mit sehr hohem Superabsorberanteil eignen. Für diesen Fall sind insbesondere Polymere mit Retentionswerten von $\geq$ 25 g/g und Permeabilitätswerte von SFC > 70 · $10^{-7}$cm$^3$ s/g erforderlich.

Die erfindungsgemäße Aufgabe wird durch die Verwerdung eines pulverförmigen, an der Oberfläche nachvernetzten, Wasser, wäßrige oder seröse Flüssigkeiten sowie Blut absorbierenden Polymerisates, aufgebaut aus

a) 55-99,9 Gew% polymerisierten, ethylenisch ungesättigten, säuregruppenenthaltenden Monomeren, die mindestens 25 Mol% neutralisiert sind,

b) 0-40 Gew% polymerisierten, ethylenisch ungesättigten, mit a) copolymerisierbaren Monomeren,

c) 0,1 - 5,0 Gew% eines oder mehrerer polymerisierter Vernetzer,

d) 0-30 Gew% eines wasserlöslichen Polymeren

wobei die Summe der Gewichtsmengen a) bis d) 100 Gew.% beträgt, dadurch gekennzeichnet, daß das Polymerisat mit

e) 0,01 bis 5 Gew.%, bezogen auf das Polymerisat, eines organischen Oberflächennachvernetzungsmittels, mit Ausnahme von Polyolen, in Form einer wässrigen Lösung und mit

f) 0,001 - 1,0 Gew%, bezogen auf das Polymerisat, eines Kations in Form eines in einer wäßrigen Lösung gelösten Salzes beschichtet und unter Erhitzen nachvernetzt worden ist,

wobei die Gesamtmenge Wasser der Beschichtungslösung 0,5 bis 10 Gew.%, bezogen auf das Polymerisat, und das Gewichtsverhältnis des Salzes zum Nachvernetzungsmittel im Bereich von 1:0,8 bis 1:4 beträgt

und wobei

vernetzte Polyacrylsäuren, die bis zu 70 Mol% als Na-Salze vorliegen und mit einer wäßrigen Lösung enthaltend $Al_2(SO_4)_3$ · 18 $H_2O$ und 1,3-Dioxolan-2-on im Gewichtsverhältnis 1:2 bzw. 1:2,5 bzw. 1: 3,33 bzw. 1:1,666 bzw. 1:1,142 bzw. 1:1 oder mit $Al_2(SO_4)_3$ · 14 $H_2O$ und 1,3-Dioxolan-2-on im Gewichtsverhältnis 1:2 oder mit $Al_2(SO_4)_3$ · 18 $H_2O$ und Ethylenglykoldiglydidylether im Gewichtsverhältnis 1:1 oder mit Alchlorid · 6 $H_2O$ und 1,3-Dioxolan-2-on im Gewichtsverhältnis 1:1,43 oder mit Eisen III chlorid · 6 $H_2O$ und 1,3-Dioxolan-2-on im Gewichtsverhältin 1:1,43 oder Ca-azetat · Hydrat bzw. bzw. Mg-acetat · Hydrat und 1,3-Dioxolan-2-on im Gewichtsverhältnis 1:10 oberflächennachvernetzt worden sind

oder vernetzte, zu 70 Mol% als Na-Salz vorliegende Polyacrylsäure, die auf native Wachs-Maisstärke oder Polyvinylalkohol aufgepfropft ist und mit einer wäßrigen Lösung enthaltend $Al_2(SO_4)_3$ · 14 $H_2O$ und 1,3-Dioxolan-2-on im Gewichtsverhältnis 1:2 beschichtet und oberflächennachvernetzt worden sind, ausgenommen sind, als Absorptionsmittel für Wasser oder wässrige Flüssigkeiten oder Blut in Konstruktionen zur Aufnahme von Körperflüssigkeiten

und wobei

der Anteil des Polymerisates in bestimmten Bereichen der Konstruktionen zwischen 60 und 100 Gew.-% liegt, gelöst.

Eine weitere Aufgabe war es Hygieneartikel bereitzustellen umfassend eine flüssigkeitsabsorbierende Sauglage beinhaltend diese pulverförmige, an der Oberfläche nachvernetzte, Wasser, wässrige oder seröse Flüssigkeiten sowie Blut absorbierende Polymerisate.

Überraschenderweise ergibt sich nämlich durch die Beschichtung eines teilchenförmigen absorbierenden Polymerisates mit einer wässrigen Lösung eines organischen Vernetzungsmittels mit Ausnahme von Polyolen, das mit den oberflächennahen Molekülgruppen, vorzugsweise mit den Carboxylgruppen, in Anwesenheit eines Kations einer Salzkomponente, vorzugsweise unter Erhitzung auf > 150 bis 250 °C reagiert hat, ein superabsorberierenden Polymerisats mit einer signifikanten Verbesserung der Permeabilitätseigenschaften bei sehr gutem Retentionsvermögen.

Völlig unerwartet führt die wässrige Lösung dieser Kombination von Nachvernetzer-Komponenten zum erwünschten Ergebnis, nämlich Superabsorberharzen mit einem hohen Retentionsvermögen auch unter Druck bei gleichzeitig ausgezeichneten Permeabilitätseigenschaften. Eine aufeinander folgende separate Anwendung sowohl einer wässrigen Lösung des organischen Nachvernetzungsmittels bzw. der wässrigen Salzlösung mit jeweiligem Erhitzen führt nicht zu einer vergleichbar guten Produktcharakteristik.

Die alleinige Verwendung von organischen Nachvernetzungsmitteln, wie beispielsweise von Alkylencarbonaten in wässriger Lösung, oder die Kombination organischer Nachvernetzer führt zu Produkten mit hoher Retentionskapazität, hoher Gelstärke und hohem Aufnahmevermögen unter Druck. Eine signifikante Steigerung der Permeabilität im gequollenen Zustand kann allerdings nur durch einen entsprechend höheren Grad der Vernetzung der Polymere bei der Polymerisation, bzw. einer stärkeren Nachvernetzung (erhöhte Mengen Nachvernetzungsmittel oder drastischere Bedingungen) und dem damit verbundenen Verlust an Retentionskapazität erreicht werden.

Die alleinige Nachvernetzung mit Kationen hoher positiver Ladungsdichte führt ebenfalls nicht zu Polymerisaten mit der erwünschten Eigenschaftskombination. Insbesondere lassen sich keine befriedigenden Werte bei der Flüssigkeitsaufnahme unter Druck und keine guten Permeabilitätseigenschaften erreichen. Eine Verbesserung der Druckstabilität oder darüber hinaus der Flüssigkeitstransporteigenschaften im gequollenen Zustand wird nicht erreicht. Die geforderten Eigenschaften können auch nicht durch kleine Mengen organischer Nachvernetzer und große Mengen Kationen erlangt werden.

Erfindungsgemäß werden als organische Nachvernetzer-Komponente e) vorzugsweise organische mindestens bifunktionelle Verbindungen mit Ausnahme von Polyolen eingesetzt, die mit den Oberflächen COOH-Gruppen des Polymerisats reagieren. Beispielsweise sind dies Alkylencarbonate, vorzugsweise mit $C_4$-$C_{10}$, besonders bevorzugt mit $C_4$- $C_6$ im Ring wie1,3-Dioxolan-2-on, 4-Methyl-1,3-dioxolan-2-on, 4,5-Dimethyl-1,3-dioxolan-2-on, 4,4-Dimethyl-1,3-dioxolan-2-on, 4-Ethyl-1,3-dioxolan-2-on, 4-Hydroxymethyl-1,3-dioxolan-2-on, 1,3-Dioxan-2-on, 4-Methyl-1,3-dioxan-2-on, 4,6-Dimethyl-1,3-dioxan-2-on oder 1,3-Dioxepan-1-on, 1,3-Dioxolane, 1,3-Dioxane, wobei 1,3-Dioxolan-2-on oder 4-Methyl-1,3-dioxolan-2-on bevorzugt ist.

Weiterhin können als Nachvernetzer-Komponente e) eingesetzt werden:
Aminoalkohole, vorzugsweise aliphatische Aminoalkohole, bevorzugt mit $C_2$-$C_{10}$ wie z.B. Diethanolamin, Triethanolamin. Weitere geeignete, aber aufgrund ihres toxikologischen Potentials als kritisch anzusehende organischen Nachvernetzerverbindungen sind: Polyepoxide, wie Polyepoxid-Ether oder Ester von polyfunktionellen, vorzugsweise difunktionellen Polyolen oder Carbonsäuren, z.B. Ethylenglycoldiglycidylether, Polyethylenglycoldiglycidylether, Glycerinpolyglycidylether Polyglycerinpolyglycidylether, Propylengylcoldiglycidylether, Polypropylenglycoldiglycidylether, Neopentylglykoldiglycidylether, Pentaerythritpolyglycidylether, Hexandioldiglycidylether, Trimethylolpropanpolyglycidylether, Sorbitolpolyglycidylether, Phthalsäurediglycidylester, Adipinsäurediglycidylester, 1,4-Phenylen-bis(2-oxazolin), Glycidol; Polyisocyanate, vorzugsweise Diisocyanate wie beispielsweise 2,4-Toluoldiisocyanat und Hexamethylendiisocyanat; Halogenepoxide wie beispielsweise Epichlor- und Epibromhydrin und $\alpha$-Methylepichlorhydrin, aliphatische Polyaminverbindungen, wie beispielsweise Ethylendiamin, Diethylentriamin, Triethylentetramin, Polyallylamin oder Polyethylenimin. Ferner sind als Nachvernetzerverbindungen Polyoxazolinverbindungen, wie beispielsweise 1,2-Ethylenbisoxazolin sowie Oxazolidinone, wie beispielsweise N-Acyl-2-Oxazolidinone sowie 2-Oxo-tetrahydro-1,3-oxazine, einsetzbar.
Die organische Nachvernetzerkomponente bzw. deren Mischungen werden in Mengen von 0,01 - 5 Gew.%, bevorzugt 0,1 - 2,5 Gew.% und besonders bevorzugt von 0,5 bis 1,5 Gew.%, bezogen auf das an seiner Oberfläche zu vernetzende Polymerisat, eingesetzt.

Unter den zuvor genannten organischen Nachvernetzerkomponenten weren die Alkylencarbonate besonders bevorzugt eingesetzt.

Als Komponente f) werden vorzugsweise wässrige Lösungen von wasserlöslichen Salzen zur Vernetzung der oberflächennahen Carboxylatgruppen eingesetzt, die als Anionen Chloride, Bromide, Sulfate, Carbonate, Nitrate, Phosphate oder organische Anionen wie Acetate und Lactate aufweisen. Die Kationen sind ein- und mehrwertigen Kationen, die sich von Alkalimetallen, wie Kalium, Natrium, Lithium, vorzugsweise Lithium ableiten. Erfindungsgemäß verwendete zweiwertige Kationen leiten sich von Zink, Beryllium, Erdalkalimetallen wie Magnesium, Calcium, Strontium ab, wobei Magnesium bevorzugt wird. Weitere Beispiele für erfindungsgemäß bevorzugt zum Einsatz kommenden drei- und höherwertige Kationen sind Kationen von Aluminium-, Eisen-, Chrom-, Mangan-, Titan-, Zirkonium- und andere Übergangsmetalle sowie von Doppelsalzen solcher Kationen oder Mischungen der genannten Salze. Bevorzugt werden drei- und höherwertige Kationen und von diesen insbesondere Aluminiumsalze und Alaune und deren unterschiedliche Hydrate wie z.B. $AlCl_3$ x 6 $H_2O$, $NaAl(SO_4)_2$ x 12 $H_2O$, $KAl(SO)_4$ x 12 $H_2O$ oder $Al_2(SO_4)_3$ x 14 -18 $H_2O$ oder $Al(NO_3)_3$ x 9 $H_2O$ eingesetzt. Besonders bevorzugt werden $Al_2(SO_4)_3$ oder $Al(NO_3)_3$ und ihre Hydrate verwendet. Eingesetzt wird die Salzkomponente, berechnet auf das Kation, in Mengen von 0,001 - 1,0 Gew.%, bevorzugt 0,005 - 0,5 Gew.%, und besonders bevorzugt 0,01 - 0,2 Gew.%, bezogen auf das Polymerisat. Das bevorzugte Gewichtsverhältnis von wasserlöslichem Salz zum Nachvernetzungsmittel beträgt 1:1 bis 1:3,5, besonders bevorzugt 1:1,2 bis 1:2,5.

Besonders bevorzugt wird eine Kombination aus dreiwertigen Kationen, vorzugsweise $Al^{3+}$ in Kombination mit Alkylencarbonaten, vorzugsweise 1,3-Dioxolan-2-on eingesetzt.

Das wasserabsorbierende Polymerisat, das oberflächenvernetzt wird, wird durch Polymerisation von a) 55-99,9 Gew% eines einfach ungesättigten Monomeren mit Säuregruppen erhalten. Hierbei sind carboxylgruppenhaltige Monomere bevorzugt, wie z.B. Acrylsäure, Methacrylsäure oder 2-Acrylamido-2-methylpropansulfonsäure oder Mischungen dieser Monomeren. Es ist bevorzugt, daß mindestens 50 Gew.% und besonders bevorzugt mindestens 75 Gew.% der Säuregruppen CarboxylGruppen sind. Die Säuregruppen sind zu mindestens zu 25 Mol% neutralisiert, d.h. liegen als Natrium-, Kalium- oder Ammoniumsalze vor. Bevorzugt liegt der Neutralisationsgrad bei mindestens 50 Mol%. Besonders bevorzugt ist ein Polymerisat, das durch Polymerisation von Acrylsäure oder Methacrylsäure, deren Carboxylgruppen zu 50-80 Mol% neutralisiert ist, in Gegenwart von Vernetzern erhalten wurde.

Als weitere Monomere b) können für die Herstellung der absorbierenden Polymerisate 0-40 Gew% ethylenisch ungesättigte mit a) copolymerisierbarer Monomere, wie z. B. Acrylamid, Methacrylamid, Hydroxyethylacrylat, Dimethylaminoalkyl(meth)-acrylat, Dimethylaminopropylacrylamid oder Acrylamidopropyltrimethylammoniumchlorid verwendet werden. Über 40 Gew% dieser Monomerern können die Quellfähigkeit der Polymerisate verschlechtern.

Als Vernetzerkomponente c), die während der Polymerisation von a) und b) vorhanden ist, können alle Verbindungen verwendet werden, die mindestens zwei ethylenisch ungesättigte Doppelbindungen oder eine ethylenisch ungesättigte Doppelbindung und eine gegenüber Säuregruppen der Monomeren a) reaktive funktionelle Gruppe oder mehrere gegenüber Säuregruppen reaktive funktionelle Gruppen tragen. Beispielhaft seien genannt: aliphatische Amide wie z. B. das Methylenbisacryl- bzw. -methacrylamid oder Ethylenbisacrylamid, ferner aliphatische Ester von Polyolen oder alkoxylierten Polyolen mit ethylenisch ungesättigten Säuren, wie Di(meth)acrylate oder Tri(meth)acrylate, Butandiol- oder Ethylenglykol, Polyglykolen, Trimethylolpropan, Di- und Triacrylatester des, vorzugsweise mit 1 bis 30 Mol Alkylenoxid oxalkylierten, vorzugsweise ethoxylierten Trimethylolpropans, Acrylat- und Methacrylatester von Glycerin und Pentaerythrit, sowie des mit vorzugsweise 1 bis 30 Mol Ethylenoxid oxethylierten Glycerins und Pentaerythrits, ferner Allylverbindungen wie Allyl(meth)acrylat, alkoxyliertes Allyl(meth)acrylat mit vorzugsweise 1 bis 30 Mol Ethylenoxid umgesetzt, Triallylcyanurat, Triallylisocyanurat, Maleinsäurediallylester, Polyallylester, Tetraallyloxiethan, Triallylamin, Tetraallylethylendiamin, Allylester der Phosphorsäure bzw. phosphorigen Säure, ferner vernetzungsfähige Monomere, wie N-Methylolverbindungen von ungesättigten Amiden wie von Methacrylamid oder Acrylamid und die davon abgeleiteten Ether. Mischungen der genannten Vernetzer können ebenfalls eingesetzt werden. Der Anteil an den vernetzenden Comonomeren liegt bei 0,1 bis 5 Gew%, bevorzugt bei 0,01 bis 3,0 Gew%, bezogen auf die Gesamtmenge der Monomeren.

Als wasserlösliche Polymere d) können in den absorbierenden Polymerisaten 0-30 Gew.% wasserlösliche Polymerisate, wie teil- oder vollverseifter Polyvinylacetate, Polyvinylpyrrolidon, Stärke oder Stärkederivate, Polyglykole oder Polyacrylsäuren enthalten, vorzugsweise einpolymerisiert sein. Das Molekulargewicht dieser Polymeren ist unkritisch, solange sie wasserlöslich sind. Bevorzugte wasserlösliche Polymere sind Stärke und Polyvinylalkohol. Der bevorzugte Gehalt an solchen wasserlöslichen Polymeren im erfindungsgemäß absorbierenden Polymerisat liegt bei 0-30 Gew.%, vorzugsweise 0-5 Gew%, bezogen auf die Gesamtmenge der Komponenten a) bis d). Die wasserlöslichen Polymere, vorzugsweise synthetische wie Polyvinylalkohol, können auch als Pfropfgrundlage für die zu polymerisierenden Monomeren dienen.

Zur Initiierung der radikalischen Polymerisation werden die gebräuchlichen Initiatoren wie z.B. Azo- oder Peroxoverbindungen, Redoxsysteme oder UV-Initiatoren (Sensibilisatoren) verwendet.

Die Herstellung der Polymerisate erfolgt vorzugsweise nach zwei Methoden:

Nach der ersten Methode wird das teilneutralisierte Monomere a), vorzugsweise die Acrylsäure in wäßriger Lösung in Gegenwart von Vernetzern und ggf. weiteren Komponenten durch radikalische Polymerisation in ein Gel überführt, das zerkleinert, getrocknet, gemahlen und auf die gewünschte Partikelgröße abgesiebt wird. Diese Lösungspolymerisation kann kontinuierlich oder diskontinuierlich durchgeführt werden. Der Stand der Technik weist ein breites Spektrum an Variationsmöglichkeiten hinsichtlich der Konzentrationsverhältnisse, Temperaturen, Art und Menge der Initiatoren aus. Typische Verfahren sind in den folgenden Veröffentlichungen beschrieben: US 4 286 082, DE 27 06 135 und US 4 076 663, deren entsprechende Offenbarung hiermit als Referenz eingeführt wird.

Auch die inverse Suspensions- und Emulsionspolymerisation kann zur Herstellung der Produkte angewendet werden. Gemäß diesen Prozessen wird eine wäßrige, teilneutralisierte Lösung der Monomeren a), vorzugsweise Acrylsäure mit Hilfe von Schutzkolloiden und/oder Emulgatoren in einem hydrophoben, organischen Lösungsmittel dispergiert und durch Radikalinitiatoren die Polymerisation gestartet. Die Vernetzer sind entweder in der Monomerlösung gelöst und werden mit dieser zusammen dosiert oder aber separat und gegebenenfalls während der Polymerisation zugefügt. Gegebenenfalls erfolgt die Zugabe eines wasserlöslichen Polymeren d) als Pfropfgrundlage über die Monomerlösung oder durch direkte Vorlage in die Ölphase. Anschließend wird das Wasser azeotrop aus dem Gemisch entfernt und das Polymerisat abfiltriert und ggf. getrocknet. Die Vernetzung kann durch Einpolymerisation eines in der Monomerenlösung gelösten polyfunktionellen Vernetzers und/oder durch Reaktion geeigneter Vernetzungsmittel mit funktionellen Gruppen des Polymeren während der Polymerisationsschritte erfolgen. Die Verfahren sind beispielsweise in den Veröffentlichungen US 43 40 706, DE 3713 601, DE 28 40 010 und WO 96/05234 beschrieben, deren entsprechende Offenbarung hiermit als Referenz eingeführt wird.

Die Trocknung des Polymerisatgels erfolgt bis zu einem Wassergehalt von 0,5-25 Gew.%, vorzugsweise von 1 bis 10 Gew.%, besonders bevorzugt 1 bis 8 Gew.% bei Temperaturen, die üblicherweise im Bereich von 100 - 200 °C liegen.

Hinsichtlich der Teilchenform des absorbierenden Polymerisaten gibt es keine besonderen Einschränkungen. Das Polymerisat kann in Form von Kügelchen vorliegen, die durch inverse Suspensionspolymerisation erhalten wurden, oder in Form von unregelmäßig geformten Teilchen, die durch Trocknung und Pulverisierung der Gelmasse aus der Lösungspolymerisation stammen. Die Teilchengröße liegt normalerweise unter 3000 $\mu$m, bevorzugt zwischen 20 und 2000 $\mu$m, und besonders bevorzugt zwischen 150 und 850 $\mu$m.

Die Nachvernetzerkomponenten werden in Form ihrer wässrigen Lösungen aufgebracht. Geeignete Lösungsmittel sind Wasser und ggf. polare, mit Wasser mischbare organische Lösungsmittel wie beispielsweise Aceton, Methanol, Ethanol oder 2-Propanol bzw. deren Gemische. Der Begriff wäßrige Lösung im Sinne der Erfindung bedeutet in Bezug auf die Lösungsmittelkomponente, daß neben dem Wasser auch noch andere organische Lösungsmittel enthalten sein können. Die Konzentration der jeweiligen Nachvernetzerkomponente in dem wässrigen Lösungsmittel kann in weiten Grenzen schwanken und liegt im Bereich von 1 bis 80 Gew.%, vorzugsweise im Bereich von 5 bis 65 Gew.% und ganz besonders bevorzugt in einem Bereich von 10 bis 40 Gew.%. Das bevorzugte Lösungsmittel für das organische Nachvernetzungsmittel bzw. die Salzkomponente ist Wasser, das in einer Menge von 0,5 - 10 Gew.%, bevorzugt 0,75 - 5 Gew.% und besonders bevorzugt 1,0 - 4 Gew.%, bezogen auf das Polymerisat verwendet wird.

Abhängig von der Löslichkeit der beiden Komponenten e) und f) wird die Lösung vor dem Aufbringen auf das Polymerisat auf 20-100 °C, bevorzugt auf 20-60 °C erwärmt. Ein getrenntes, aber gleichzeitiges Zudosieren von einer Lösung des organischen Nachvernetzers und einer Lösung der Salzkomponente ist ebenfalls möglich, wenn eine homogene Verteilung beider Komponenten auf dem Polymerisat gewährleistet ist und das Material anschließend thermisch nachbehandelt wird. Bevorzugt ist das Aufbringen einer einzigen wäßrigen Lösung auf das Polymerisat, in der beide Komponenten gelöst sind.

Die Nachvernetzerlösung sollte sehr gut mit den Polymerteilchen vermischt werden. Geeignete Mischaggregate zum Aufbringen der Nachvernetzerlösung sind z.B. Patterson-Kelley-Mischer, DRAIS-Turbulenzmischer, Lödigemischer, Ruberg-Mischer, Schneckenmischer, Tellermischer und Wirbelschichtmischer sowie kontinuierlich arbeitende senkrechte Mischer, in denen das Polymerisat-Pulver mittels rotierender Messer in schneller Frequenz gemischt wird (Schugi-Mischer). Es besteht auch die Möglichkeit, die Beschichtung des Polymerisates während eines Verfahrensschrittes bei der Herstellung des Polymerisates vorzunehmen. Hierzu ist besonders der Prozeß der inversen Suspensionspolymerisation geeignet.

Nachdem die Nachvernetzerlösung mit den Polymerteilchen vermischt worden ist, erfolgt die Nachvernetzungsreaktion vorzugsweise bei Temperaturen im Bereich von > 150°C bis 250°C, bevorzugt von 160°C bis 220°C und besonders bevorzugt von 170°C bis 200°C. Die optimale Zeitdauer der Nacherhitzung kann für die einzelnen Vernetzertypen mit wenigen Versuchen leicht ermittelt werden. Sie wird dadurch begrenzt, wenn das gewünschte Eigenschaftsprofil des Superabsorbers infolge von Hitzeschädigung wieder zerstört wird. Die thermische Behandlung kann in üblichen Trocknern oder Öfen durchgeführt werden; beispielhaft seien Drehrohröfen, Wirbelbetttrockner, Tellertrockner, Paddeltrockner oder Infrarottrockner genannt.

Die Polymeren können in großtechnischer Weise nach bekannten kontinuierlich oder diskontinuierlich hergestellt werden.

Die Polymerisate können für weite Anwendungsgebiete eingesetzt werden. Wenn sie z. B. als Absorbierungsmittel in Damenbinden, Windeln oder in Wundabdeckungen verwendet werden, besitzen sie die Eigenschaft, daß sie große Mengen an Menstrationsblut, Urin oder anderen Körperflüssigkeiten schnell absorbieren. Da die Absorbierungsmittel die absorbierten Flüssigkeiten auch unter Druck zurückhalten und zusätzlich in der Lage sind, im gequollenen Zustand weitere Flüssigkeit innerhalb der Konstruktion zu verteilen, werden sie besonders bevorzugt in höheren Konzentrationen, in Bezug auf das hydrophile Fasermaterial wie z.B. Fluff eingesetzt als dies bisher möglich war. Sie eignen sich auch für den Einsatz als homogene Superabsorberschicht ohne Fluffanteil innerhalb der Windelkonstruktion, wodurch besonders dünne Windeln möglich sind. Weiterhin eignen sich die Polymere zum Einsatz in Hygieneartikel (Inkontinenzprodukte) für Erwachsene.

Solche absorbierende Hygieneprodukte besitzen in der Regel einen allgemeinen Aufbau aus einer körperzugewandten flüssigkeitsdurchlässigen Abdeckung (1), einer flüssigkeitsabsorbierenden Sauglage (2) sowie einer im wesentlichen flüssigkeitsundurchlässigen, körperabgewandten Außenschicht (3). Optional finden auch weitere Konstruktionen zur schnellen Aufnahme und Verteilung von Körperflüssigkeit (4) im Saugkern Anwendung. Diese Konstruktionen werden häufig, aber nicht zwingend zwischen der körperzugewandten flüssigkeitsdurchlässigen Abdeckung (1) und der flüssigkeitsabsorbierenden Sauglage (2) eingesetzt.

Die flüssigkeitsdurchlässige Abdeckung (1) besteht in der Regel aus einem nichtgewebten, faserigen Vlies oder einer anderen porösen Konstruktion.

Als Materialien für diese Abdeckung (1) kommen z. B. synthetische Polymere wie etwa Polyvinylchlorid oder -fluorid, Polytetrafluorethylen (PTFE), Polyvinylalkohole und -Derivate, Polyacrylate, Polyamide, Polyester, Polyurethane, Polystyrol, Polysiloxane oder Polyolefine (z.B. Polyethylen (PE) oder Polypropylen (PP)) sowie natürliche Fasermaterialien sowie beliebige Kombinationen aus den vorgenannten Materialien im Sinne von Mischmaterialien oder Verbundmaterialien oder Copolymerisaten in Frage.

Die flüssigkeitsdurchlässige Abdeckung (1) hat hydrophilen Charakter. Sie kann zudem aus einer Kombination von hydrophilen und hydrophoben Bestandteilen bestehen. Bevorzugt ist in der Regel eine hydrophile Ausrüstung der flüssigkeitsdurchlässigen Abdeckung (1), um schnelle Einsickerzeiten von Körperflüssigkeit in die flüssigkeitsabsorbierende Sauglage (2) zu ermöglichen, jedoch werden auch partiell hydrophobierte Abdeckungen (1) verwendet.

Die flüssigkeitsabsorbierende Sauglage (2) enthält die superabsorbierenden Pulver bzw. Granulate und ggf. weitere Komponenten aus beispielsweise faserigen Materialien, schaumförmigen Materialien, filmbildenden Materialien oder porösen Materialien sowie Kombinationen von zwei oder mehreren dieser Materialien. Jedes dieser Materialien kann entweder natürlichen oder synthetischen Ursprungs sein oder durch chemische oder physikalische Modifikation von natürlichen Materialien hergestellt worden sein. Die Materialien können hydrophil oder hydrophob sein, wobei hydrophile Materialien bevorzugt sind. Dies gilt insbesondere für solche Zusammensetzungen, die ausgeschiedene Körperflüssigkeiten effizient aufnehmen und in Richtung zu weiter von der Eintrittsstelle der Körperflüssigkeit entfernte Regionen des absorbierenden Kerns transportieren sollen.

Als hydrophile Fasermaterialien sind geeignet z.B. Cellulosefasern, modifizierte Cellulosefasern (z.B. versteifte Cellulosefasern), Polyesterfasern (z.B. Dacron) hydrophiles Nylon oder aber auch hydrophilisierte hydrophobe Fasern, wie z. B. mit Tensiden hydrophilisierte Polyolefine (PE, PP), Polyester, Polyacrylate, Polyamide, Polystyrol, Polyurethane und andere.

Bevorzugt werden Cellulosefasern und modifizierte Cellulosefasern eingesetzt. Kombinationen von Cellulosefasern und/oder modifizierten Cellulosefasern mit synthetischen Fasern wie z. B. PE/PP Verbundmaterialien, sogenannte Bikomponentenfasern, wie sie z.B. zur Thermobondierung von Airlaidmaterialien verwendet werden oder anderen Materialien sind ebenfalls gebräuchlich.

Die Fasermaterialien können in verschiedenen Anwendungsformen vorliegen, z.B. als lose aus einem Luftstrom oder aus wässriger Phase abgeschiedene oder abgelegte Cellulosefasern, als nichtgewebtes Vlies oder als Tissue. Kombinationen verschiedener Anwendungsformen sind möglich.

Optional können neben den superabsorbierenden Polymerisaten weitere pulverförmige Substanzen eingesetzt werden, wie z.B. geruchsbindende Substanzen wie Cyclodextrine, Zeolithe, anorganische oder organische Salze und ähnliche Materialien.

Als poröse Materialien und schaumförmige Materialien können z.B. Polymerschäume eingesetzt werden wie sie in den Schriften DE 44 18 319 A1 und DE 195 05 709 A1 beschrieben sind.

Zur mechanischen Stabilisierung der flüssigkeitsabsorbierenden Sauglage (2) können thermoplastische Fasern (Z.B. Bikomponentenfasern aus Polyolefinen), Polyolefingranulate, Latexdispersionen oder Heisskleber verwendet werden. Optional werden eine oder mehrere Lagen Tissue zur Stabilisierung verwendet.

Die flüssigkeitsabsorbierende Sauglage (2) kann einlagig sein oder aus mehreren Schichten bestehen. Dazu können Konstruktionen verwendet, die aus hydrophilen Fasern, bevorzugt Cellulosefasern, optional einer Konstruktion zur schnellen Aufnahme und Verteilung von Körperflüssigkeit (4) wie zum Beispiel chemisch versteifte (modifizierte) Cellulosefasern oder Highloftvliese aus hydrophilen oder hydrophilisierten Fasern sowie superabsorbierenden Polymeren bestehen.

Die superabsorbierenden Polymerisate können dabei homogen in den Cellulosefasern oder den versteiften Cellulosefasern verteilt sein, sie können auch lagig zwischen den Cellulosefasern oder den versteiften Cellulosefasern eingebracht sein, oder die Konzentration der superabsorbierenden Polymerisate kann innerhalb der Cellulosefasern oder versteiften Cellulosefasern einen Gradienten aufweisen. Das Verhältnis der Gesamtmenge an superabsorbierendem Polymer und der Gesamtmenge an Cellulosefasern oder den versteiften Cellulosefasern im absorbierenden Saugkern kann zwischen 0 - 100 Gew.% variieren, wobei in einer Ausführungsform lokal, z. B. bei Gradienteneintrag oder schichtweisem Eintrag Konzentrationen von bis zu 100 % superabsorbierende Polymerisate möglich sind. Derartige Konstruktionen mit Bereichen hoher Konzentrationen von absorbierendem Polymerisate, wobei der Anteil des Polymerisates in bestimmten Bereichen zwischen 60 und 100 Gew.%, bevorzugt zwischen 90 und 100 Gew.% liegt, sind beispielsweise auch in der Patentschrift US 5,669,894 beschrieben.

Optional können auch mehrere verschiedene superabsorbierende Polymerisate, die sich zum Beispiel in der Sauggeschwindigkeit, der Permeabilität, der Speicherkapazität, der Absorption gegen Druck, der Kornverteilung oder auch der chemischen Zusammensetzung gleichzeitig eingesetzt werden. Die verschiedenen Superabsorber können miteinander vermischt in das Saugkissen eingebracht werden oder aber lokal differenziert im Absorbent Core plaziert werden. Eine solche differenzierte Plazierung kann in Richtung der Dicke des Saugkissens oder der Länge oder Breite des Saugkissens erfolgen.

In der flüssigkeitsabsorbierenden Sauglage (2) befindet sich eine oder mehrere der superabsorbierenden Polymerisate enthaltenden Lagen ggf. mit Cellulosefasern oder versteiften Cellulosefasern . In einer bevorzugten Ausführungsform werden Konstruktionen aus Kombinationen von Lagen mit homogenem Superabsorbereintrag und zusätzlich schichtweiser Einbringung verwendet.

Ggf. können die Absorptionsartikel weitere Lagen von reinen Cellulosefasern oder versteiften Cellulosefasern an der körperzugewandten Seite und / oder auch der körperabgewandten Seite aufweisen.

Die oben beschriebenen Aufbaumöglichkeiten können sich auch mehrfach wiederholen, wobei es sich um eine Aufeinanderschichtung zweier oder mehrerer gleicher Lagen oder aber auch um Aufeinanderschichtung zweier oder mehrerer unterschiedlicher Konstruktionen unterschiedlichen Aufbaus handeln kann. Dabei liegen die Unterschiede in wiederum rein konstruktiver Art oder aber im Typ des verwendeten Materials, wie z.B. die Verwendung von erfindungsgemäßen absorbierenden Polymerisaten oder mit anderen Polymerisaten, aber verschiedener Zellstoffarten.

Ggf. kann das gesamte Saugkissen oder aber auch einzelne Lagen der flüssigkeitsabsorbierenden Sauglage (2) durch Lagen von Tissue von anderen Komponenten des Absorptionsartikels getrennt oder stehen in direktem Kontakt mit anderen Lagen oder Komponenten sein.

Exemplarisch können zum Beispiel die Konstruktion zur schnellen Aufnahme und Verteilung von Körperflüssigkeit (4) und die flüssigkeitsabsorbierende Sauglage (2) durch Tissue voneinander getrennt sein oder aber in direktem Kontakt miteinander stehen. Sofern keine separate Konstruktion zur schnellen Aufnahme und Verteilung von Körperflüssigkeit (4) zwischen der flüssigkeitsabsorbierenden Sauglage (2) und der körperzugewandten flüssigkeitsdurchlässigen Abdeckung (1) existiert, sondern der Effekt der Flüssigkeitsverteilung z.B durch die Verwendung einer speziellen körperzugewandten flüssigkeitsdurchlässigen Abdeckung (1) erreicht werden soll, kann die flüssigkeitsabsorbierende Sauglage (2) ebenfalls optional von der körperzugewandten flüssigkeitsdurchlässigen Abdeckung (1) durch ein Tissue getrennt sein.

Statt Tissue kann optional auch nichtgewebtes Vlies in die flüssigkeitsabsorbierende Sauglage (2) eingebracht werden. Beide Komponenten führen zu dem erwünschten Nebeneffekt der Stabilisierung und Festigung des Absorptionskerns im feuchten Zustand.

Die flüssigkeitsabsorbierende Sauglage, insbesondere faserhaltige, superabsorbierende Polymerisate haltige, flüssigkeitsverteilende und -speichernde Schichten lassen lassen sich nach einer Vielzahl von Herstellverfahren erzeugen.

Neben den etablierten konventionellen Prozessen, wie die sich unter Drumforming mit Hilfe von Formrädern, -taschen und Produktformen und entsprechend angepassten Dosiereinrichtungen für die Rohstoffe zusammengefaßt werden können, sind moderne etablierte Verfahren wie der Airlaidprozess (z.B. EP 850 615, Sp. 4 Zeile 39 bis Sp. 5 Zeile 29, US 4.640.810) mit allen Formen der Dosierung, Ablage der Fasern und Verfestigung wie Hydrogenbonding (z.B. DE 197 50 890, Sp. 1 Zeile 45 bis Sp. 3 Zeile 50, Thermobonding, Latexbonding (z.B. EP 850 615, Sp. 8 Zeile 33 bis Sp. 9 Zeile 17 und Hybridbonding, der Wetlaid Prozeß (z.B. PCT WO 99/49905, Sp. 4 Zeile 14 bis Sp. 7 Zeile 16), Carding-, Meltblown-, Spunblown-Prozesse sowie ähnliche Prozesse zur Herstellung von superabsorberhaltigen Non-Wovens (im Sinne der der Definition der EDANA, Brüssel) auch in Kombinationen dieser Verfahren mit- und untereinander übliche Methoden zur Herstellung von den o.g. Flüssigkeitsspeichern zu verstehen.

Als weitere Herstellungsverfahren kommen die Herstellung von Laminaten im weitesteten Sinne sowie von extrudierten und coextrudierten, naß- und trockensowie nachträglich verfestigten Strukturen in Frage.

Eine Kombinationen dieser Verfahrensmöglichkeiten mit- und untereinander ist ebenfalls möglich.

Für die Herstellung von Absorptionsartikeln mit einer schnellen Aufnahme und Verteilung von Körperflüssigkeit (4) können zusätzlich zum Beispiel chemisch versteiften (modifizierte) Cellulosefasern oder Highloftvliesen aus hydrophilen oder hydrophilisierten Fasern oder einer Kombination von beidem mitverwendet werden.

Chemisch versteifte, modifizierte Cellulosefasern können zum Beispiel erzeugt werden aus Cellulosefasern, die durch Vernetzer wie z.B. $C_2$ - $C_8$ Dialdehyde, $C_2$ - $C_8$ Monoaldehyde mit einer zusätzlichen Säurefunktion oder $C_2$ - $C_9$ Polycarbonsäuren in einer chemischen Reaktion umgesetzt werden. Spezielle Beispiele sind: Glutaraldehyd, Glyoxal, Glyoxalsäure oder Zitronensäure. Ebenfalls bekannt sind kationisch modifizierte Stärke oder Polyamid-Epichlorhydrinharze (z. B. KYMENE 557H, Hercules Inc., Wilmington , Delaware). Durch die Vernetzung wird eine verdrehte, gekräuselte Struktur erreicht und stabilisiert, die sich vorteilhaft auf die Geschwindigkeit der Flüssigkeitsaufnahme auswirkt.

Die absorbierenden Hygieneprodukte können in ihrem Flächengewicht und Dicke und damit der Dichte stark variieren. Typischerweise liegen die Dichten der Bereiche der Absorptionskerne zwischen 0,08 und 0,25 $g/cm^3$. Die Flächengewichte liegen zwischen 10 und 1000 $g/m^2$, wobei bevorzugt Flächengewichte zwischen 100 und 600 $g/m^2$ realisiert werden (siehe auch US 5,669,894). Die Dichte variiert in der Regel über die Länge des absorbierenden Kerns. Dies tritt als Folge einer gezielten Dosierung der Cellulosefaser- oder versteiften Cellulosefasermenge oder der Menge des superabsorbierenden Polymerisats ein, da diese Komponenten in bevorzugten Ausführungsformen stärker in den Frontbereich des absorbierenden Einwegartikels eingebracht werden.

Die erfindungsgemäßen Superabsorber zeigen überraschenderweise eine bedeutende Verbesserung der Permeabilität, d. h. eine Verbesserung des Flüssigkeitstransportes im gequollenen Zustand. Es werden Polymerisate mit Permeabilitäts-Werten (SFC) von bis zu 70 · $10^{-7}$ $cm^3$ s /g bei einer Retention (TB) von mindestens 27 g/g erhalten, vorzugsweise Polymere mit SFC-Werten von > 70 · $10^{-7}$ bis ≥ 150 · $10^{-7}$ $cm^3$ s/g bei einer Retention (TB) von mindestens 25 g/g. Neben diesen ausgezeichneten SFC- und Retentionswerten zeigen die erfindungsgemäßen Polymere Meßwerte für die Flüssigkeitsaufnahme unter Druck (AAP 0,7) von mindestens 18 g/g.

Die erfindungsgemäßen Produkte mit dieser hervorragenden Eigenschaftskombination aus sehr hohen SFC-Werten, hoher Retention und hoher Absorption unter Druck können ohne die Verwendung toxikologisch bedenklicher Substanzen hergestellt werden.

**Testmethoden**

Zur Charakterisierung der erfindungsgemäßen, absorbierenden Polymerisate werden Retention (TB), Aufnahme unter Druck (AAP) und die Durchlässigkeit für 0,9%ige Kochsalzlösung im gequollenen Zustand (SFC) bestimmt.

a) Die Retention wird nach der Teebeutelmethode und als Mittelwert aus drei Messungen angegeben. Etwa 200 mg Polymerisat werden in einen Teebeutel eingeschweißt und für 30 Minuten in 0,9%ige NaCl-Lösung getaucht. Anschließend wird der Teebeutel in einer Schleuder (23 cm Durchmesser, 1.400 Upm) 3 Minuten geschleudert und gewogen. Einen Teebeutel ohne wasserabsorbierendes Polymerisat läßt man als Blindwert mitlaufen.

$$\text{Retention} = \text{Auswaage-Blindwert/Einwaage [g/g]}$$

b) Flüssigkeitsaufnahme unter Druck (AAP-Test, gemäß EP 0 339 461)

Die Aufnahme unter Druck (Druckbelastung 50 g/cm$^2$) wird nach einer in der EP 0339461, Seite 7, beschriebenen Methode bestimmt. In einen Zylinder mit Siebboden werden ca. 0,9 g Superabsorber eingewogen. Die gleichmäßig aufgestreute Superabsorberlage wird mit einem Stempel belastet, der einen Druck von 50 g/cm$^2$ ausübt. Der zuvor gewogene Zylinder wird anschließend auf eine Glasfilterplatte gestellt, die sich in einer Schale mit 0,9%iger NaCl-Lösung befindet, deren Flüssigkeitniveau genau der Höhe der Filterplatte entspricht. Nachdem man die Zylindereinheit 1 Stunde lang 0,9%ige NaCl-Lösung saugen gelassen hat, wird diese zurückgewogen und der AAP wie folgt berechnet:

$$\text{AAP} = \text{Auswaage (Zylindereinheit} + \text{Superabsorber)-Einwaage (Zylindereinheit} +$$

$$\text{vollgesogener Superabsorber) / Einwaage Superabsorber}$$

c) Permeabilität im gequollenen Zustand (SFC-Test, gemäß WO 95/22356)

In einen Zylinder mit Siebboden werden ca. 0,9 g Superabsorbermaterial eingewogen und sorgfältig auf der Siebfläche verteilt. Das Superabsorbermaterial läßt man in JAYCO synthetischen Urin [Zusammensetzung: 2,0 g Kaliumchlorid; 2,0 g Natriumsulfat; 0,85 g Ammoniumdihydrogenphosphat; 0,15 g Ammoniumhydrogenphosphat; 0,19 g Calciumchlorid; 0,23 g Magnesiumchlorid als wasserfreie Salze in 1 l destilliertem Wasser gelöst] 1 Stunde lang gegen einen Druck von 20 g/cm$^2$ quellen. Nach Erfassung der Quellhöhe des Superabsorbers läßt man bei konstantem hydrostatischem Druck 0,118 M NaCl-Lösung aus einem nivellierten Vorratsgefäß durch die gequollene Gelschicht laufen. Die gequollene Gelschicht ist während der Messung mit einem speziellen Siebzylinder abgedeckt, der eine gleichmäßige Verteilung der 0,118 M NaCl-Lösung oberhalb des Gels und konstante Bedingungen (Meßtemperatur 20-25°C) während der Messung bezüglich der Gelbettbeschaffenheit gewährleistet. Der auf den gequollenen Superabsorber wirkende Druck ist weiterhin 20 g/cm$^2$. Mit Hilfe eines Computers und einer Waage wird die Flüssigkeitsmenge, die die Gelschicht als Funktion der Zeit passiert in Intervallen von 20 Sekunden innerhalb einer Zeitperiode von 10 Minuten erfaßt. Die Fließrate g/s durch die gequollene Gelschicht wird mittels Regressionsanalyse mit Extrapolation der Steigung und Ermittlung des Mittelpunkts auf den Zeitpunkt t=0 der Fließmenge innerhalb der Minuten 2-10 ermittelt. Die Berechnung des SFC-Wertes (K) berechnet sich wie folgt:

$$K = \frac{F_s(t=0) \cdot L_o}{r \cdot A \cdot \Delta P} = \frac{F_s(t=0) \cdot L_0}{139506}$$

Wobei:

$F_s$ (t = 0)  die Fließrate in g/s
$L_0$  die Dicke der Gelschicht in cm
r  die Dichte der NaCl-Lösung (1,003 g/cm$^3$)
A  die Fläche der Oberseite der Gelschicht im Meßzylinder (28,27 cm$^2$)
$\Delta P$  der hydrostatische Druck, der auf der Gelschicht lastet (4920 dyne/cm$^2$)
und K  der SFC-Wert ist [cm$^3$ * s * g$^{-1}$]

Die formale Addition der Zahlenwerte der Teebeutelretention und des SFC-Wertes verdeutlicht den sprunghaften Anstieg dieser Eigenschaftskombination bei den erfindungsgemäßen Polymerisaten im Vergleich zu unbehandeltem Superabsorberpulver oder Produkten die nach bekannten Methoden oberflächlich nachvernetzt wurden. Der Zahlenwert wird bei den erfindungsgemäßen Produkten nicht durch einen hohen Beitrag einer der beiden Werte erreicht (z. B. eines hohen TB-Retentionswertes und eines niedrigen SFC-Wertes und umgekehrt).

**Beispiele**

In den Beispielen und Vergleichsbeispielen wurde das zur nachvernetzenden Oberflächenbehandlung jeweils eingesetzte Pulver auf eine Teilchengröße von 150 $\mu$m bis 850 $\mu$m abgesiebt.

**Beispiel 1 (Nicht erfinderungsgemäß)**

In 965,115 g einer wässrigen Lösung von Natriumacrylat mit einem Neutralisationsgrad von 70 mol% (Monomer-Konzentration: 37,7 %) werden 1,05 g Polyethylenglycol 300diacrylat und 1,35 g Polyethylenglycol(750)monoallyletheracrylat als Vernetzer gelöst. Die Monomerenlösung wird in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natrium-peroxidisulfat in 10 g dest. Wasser, 0,1 g 2,2'-Azobis-2-amidinopropandihydrochlorid in 10 g dest. Wasser, 0,07 g 35 %ige Wasserstoffperoxid lösung in 10 g dest. Wasser und 0,015 g Ascorbinsäure in 2 g dest. Wasser gestartet. Nachdem die Endtemperatur (ca. 100 °C) erreicht war, wurde das Gel mit einem Fleischwolf zerkleinert und 2 h bei 150 °C in einem Umluftofen getrocknet. Das getrocknete Produkt wurde grob zerstoßen, gemahlen und die Partikel der Größe 150 - 850 $\mu$m zur weiteren Umsetzung ausgesiebt (Pulver A).

50 g Pulver A wurden unter kräftigem Rühren mit einer Lösung 0,25 g

Aluminiumsulfat-18-Hydrat und 0,25 g Wasser und anschließend mit einer Lösung aus 0,5 g 1,3-Dioxolan-2-on und 0,5 g Wasser vermischt und danach für 60 min. in einem Ofen, der auf 170 °C temperiert war, erhitzt.
Zum Vergleich wurden 50 g Pulver A mit einer Lösung aus 0,5 g 1,3-Dioxolan-2-on und 1,25 g Wasser vermischt und anschließend für 60 min. in einem Ofen, der auf 170 °C temperiert war, erhitzt (Vergleichsbeispiel 1)

| Produkt | TB | AAP$_{0.7}$ | SFC | TB+SFC |
|---|---|---|---|---|
| | [g/g] | [g/g] | [cm$^3$ s 10$^{-7}$/g] | |
| **Pulver A** | 31,0 | | 0 | 31,0 |
| **Beispiel 1** | 28,5 | 23,8 | 70 | 98,5 |
| **Vergleichsbeispiel 1** | 28,7 | 25,0 | 20 | 48,7 |

**Beispiel 2**

50 g Pulver A wurden unter kräftigem Rühren mit einer Lösung 0,14 g

Aluminiumnitrat-9-Hydrat und 0,14 g Wasser und anschließend mit einer Lösung aus 0,5 g 1,3-Dioxolan-2-on und 0,5 g Wasser vermischt und danach für 60 min. in einem Ofen, der auf 170 °C temperiert war, erhitzt.
Zum Vergleich wurden 50 g Pulver A mit einer Lösung aus 0,5 g 1,3-Dioxolan-2-on und 1,25 g Wasser vermischt und anschließend für 60 min. in einem Ofen, der auf 170 °C temperiert war, erhitzt (Vergleichsbeispiel 1)

| Produkt | TB | AAP$_{0.7}$ | SFC | TB+SFC |
|---|---|---|---|---|
| | [g/g] | [g/g] | [cm$^3$ s 10$^{-7}$/g] | |
| **Pulver A** | 31,0 | | 0 | 31,0 |
| **Beispiel 2** | 28,5 | 24,5 | 75 | 103,5 |

**Beispiel 3**

In 965,175 g einer wässrigen Lösung von Natriumacrylat mit einem Neutralisationsgrad von 70 mol% (Monomer-Konzentration: 37,7 %) werden 0,84 g Triallylamin und 1,5 g Polyethylenglycol(750)monoallyletheracrylat als Vernetzer gelöst. Die Monomerenlösung wird in einem Kunststoff-Polymerisationsgefäß für 30 Minuten mit Stickstoff durchspült, um den gelösten Sauerstoff zu entfernen. Bei einer Temperatur von 4 °C wird die Polymerisation durch die aufeinanderfolgende Zugabe von 0,3 g Natriumperoxidisulfat in 10 g dest. Wasser, 0,1 g 2,2'-Azobis-2-amidinopropandihydrochlorid in 10 g dest. Wasser, 0,07 g 35 %ige Wasserstoffperoxidlösung in 10 g dest. Wasser und 0,015 g Ascorbinsäure in 2 g dest. Wasser gestartet. Nachdem die Endtemperatur (ca. 100 °C) erreicht war, wurde das Gel mit einem Fleichwolf zerkleinert und 2 h bei 150 °C in einem Umluftofen getrocknet. Das getrocknete Produkt wurde grob zerstoßen, gemahlen und die Partikel der Größe 150 - 850 $\mu$m zur weiteren Umsetzung ausgesiebt (Pulver B).

50 g Pulver B wurden unter kräftigem Rühren mit einer Lösung 0,25 g

Aluminiumsulfat-18-Hydrat, 0,5 g Hydroxymethyl-1,3-dioxolan-2-on und 1,25 g Wasser vermischt und danach für 30 min. in einem Ofen, der auf 180 °C temperiert war, erhitzt (Beispiel 3).

50 g Pulver B wurden unter kräftigem Rühren mit einer Lösung 0,5 g 1,3-Dioxolan-2-on, 0,05 g Ethylenglycoldiglycidylether und 1,5 g Wasser vermischt und danach für 30 min. in einem Ofen, der auf 180 °C temperiert war, erhitzt (Vergleichsbeispiel 2).

50 g Pulver B wurden unter kräftigem Rühren mit einer Lösung 0,25 g Glycerin, 0,25 g 1,3-Dioxolan-2-on und 1,5 g Wasser vermischt und danach für 30 min. in einem Ofen, der auf 180 °C temperiert war, erhitzt (Vergleichsbeispiel 3).

50 g Pulver B wurden unter kräftigem Rühren mit einer Lösung 0,25 g 1,3-Dioxolan-2-on 0,25 g Ethylendiamin und 1,5 g Wasser vermischt und danach für 30 min. in einem Ofen, der auf 180 °C temperiert war, erhitzt (Vergleichsbeispiel 4).

| Produkt | TB | $AAP_{0.7}$ | SFC | TB+SFC |
| --- | --- | --- | --- | --- |
| | [g/g] | [g/g] | $[cm^3 \, s \, 10^{-1}/g]$ | |
| **Pulver B** | 30,5 | | 0 | 30,5 |
| **Beispiel 3** | 26,0 | 22,7 | 65 | 91 |
| **Vergleichsbeispiel 2** | 26,7 | 23,4 | 37 | 63,7 |
| **Vergleichsbeispiel 3** | 26,4 | 22,8 | 42 | 68,4 |
| **Vergleichsbeispiel 4** | 26,8 | 20,6 | 16 | 42,8 |

## Patentansprüche

1. Verwendung pulverförmiger, an der Oberfläche nachvernetzter, Wasser, wässrige oder seröse Flüssigkeiten sowie Blut absorbierender Polymerisate, aufgebaut aus

a) 55-99,9 Gew.-% polymerisierten, ethylenisch ungesättigten, säuregruppenenthaltenden Monomeren, die zu mindestens 25 Mol-% neutralisiert sind,
b) 0-40 Gew.-% polymerisierten, ethylenisch ungesättigten, mit a) copolymerisierbaren Monomeren,
c) 0,1-5,0 Gew.-% eines oder mehrerer einpolymerisierter Vernetzer,
d) 0-30 Gew.-% eines wasserlöslichen Polymeren
wobei die Summe der Gewichtsmengen a) bis d) 100 Gew.-% beträgt,
wobei das Polymerisat mit
e) 0,01 bis 5 Gew. %, bezogen auf das Polymerisat, wenigstens eines organischen Oberflächennachvernetzungsmittels, mit Ausnahme von Polyolen, in Form einer wässrigen Lösung und mit
f) 0,001-1,0 Gew%, bezogen auf das Polymerisat, eines Kations in Form eines in einer wässrigen Lösung gelösten Salzes beschichtet und unter Erhitzen nachvernetzt worden ist,

wobei die Gesamtmenge Wasser der Beschichtungslösung 0,5 bis 10 Gew.%, bezogen auf das Polymerisat, beträgt und das Gewichtsverhältnis des Salzes zum Nachvernetzungsmittel im Bereich von 1 : 0,8 bis 1 : 4 liegt,
und wobei
vernetzte Polyacrylsäuren, die bis zu 70 Mol% als Na-Salze vorliegen und mit einer wäßrigen Lösung enthaltend $Al_2(SO_4)_3 \cdot 18 \, H_2O$ und 1,3-Dioxolan-2-on im Gewichtsverhältnis 1:2 bzw. 1:2,5 bzw. 1: 3,33 bzw. 1:1,666 bzw. 1: 1,142 bzw. 1:1 oder mit $Al_2(SO_4)_3 \cdot 14 \, H_2O$ und 1,3-Dioxolan-2-on im Gewichtsverhältnis 1:2 oder mit $Al_2(SO_4)_3 \cdot 18 \, H_2O$ und Ethylenglykoldiglydidylether im Gewichtsverhältnis 1:1 oder mit Alchlorid $\cdot 6 \, H_2O$ und 1,3-Dioxolan-2-on im Gewichtsverhältnis 1:1,43 oder mit Eisen III chlorid . $6 \, H_2O$ und 1,3-Dioxolan-2-on im Gewichtsverhältnis 1: 1,43 oder Caazetat $\cdot$ Hydrat bzw. bzw. Mg-acetat $\cdot$ Hydrat und 1,3-Dioxolan-2-on im Gewichtsverhältnis 1:10 oberflächennachvernetzt worden sind
oder vernetzte, zu 70 Mol% als Na-Salz vorliegende Polyacrylsäure, die auf native Wachsmaisstärke oder Polyvinylalkohol aufgepfropft ist und mit einer wäßrigen Lösung enthaltend $Al_2(SO_4)_3 \cdot 14 \, H_2O$ und 1,3-Dioxolan-2-on im Gewichtsverhältnis 1:2 beschichtet und oberflächennachvernetzt worden sind,
ausgenommen sind,
als Absorptionsmittel für Wasser oder wässrige Flüssigkeiten oder Blut in Konstruktionen zur Aufnahme von Körperflüssigkeiten, **dadurch gekennzeichnet, dass** der Anteil des Polymerisates in bestimmten Bereichen der Konstruktionen zwischen 60 und 100 Gew.-% liegt.

2. Hygieneartikel, umfassend eine flüssigkeitsabsorbierende Sauglage beinhaltend pulverförmige, an der Oberfläche nachvemetzte, Wasser, wässrige oder seröse Flüssigkeiten sowie Blut absorbierende Polymerisate, aufgebaut aus

a) 55-99,9 Gew.-% polymerisierten, ethylenisch ungesättigten, säuregruppenenthaltenden Monomeren, die zu mindestens 25 Mol-% neutralisiert sind,
b) 0-40 Gew.-% polymerisierten, ethylenisch ungesättigten, mit a) copolymerisierbaren Monomeren,
c) 0,1-5,0 Gew.-% eines oder mehrerer einpolymerisierter Vernetzer,
d) 0-30 Gew.-% eines wasserlöslichen Polymeren
wobei die Summe der Gewichtsmengen a) bis d) 100 Gew.-% beträgt,
wobei das Polymerisat mit
e) 0,01 bis 5 Gew. %, bezogen auf das Polymerisat, wenigstens eines organischen Oberflächennachvernetzungsmitteis, mit Ausnahme von Polyolen, in Form einer wässrigen Lösung und mit
f) 0,001-1,0 Gew%, bezogen auf das Polymerisat, eines Kations in Form eines in einer wässrigen Lösung gelösten Salzes beschichtet und unter Erhitzen nachvernetzt worden ist,

wobei die Gesamtmenge Wasser der Beschichtungslösung 0,5 bis 10 Gew.%, bezogen auf das Polymerisat, beträgt und das Gewichtsverhältnis des Salzes zum Nachvernetzungsmittel im Bereich von 1 : 0,8 bis 1 : 4 liegt,
und wobei
vernetzte Polyacrylsäuren, die bis zu 70 Mol% als Na-Salze vorliegen und mit einer wäßrigen Lösung enthaltend $Al_2(SO_4)_3 \cdot$ 18 $H_2O$ und 1,3-Dioxolan-2-on im Gewichtsverhältnis 1:2 bzw. 1:2,5 bzw. 1: 3,33 bzw. 1:1,666 bzw. 1: 1,142 bzw. 1:1 oder mit $Al_2(SO_4)_3 \cdot$ 14 $H_2O$ und 1,3-Dioxolan-2-on im Gewichtsverhältnis 1:2 oder mit $Al_2(SO_4)_3 \cdot$ 18 $H_2O$ und Ethylenglykoldiglydidylether im Gewichtsverhältnis 1:1 oder mit Alchlorid · 6 $H_2O$ und 1,3-Dioxolan-2-on im Gewichtsverhältnis 1:1,43 oder mit Eisen III chlorid · 6 $H_2O$ und 1,3-Dioxolan-2-on im Gewichtsverhältnis 1: 1,43 oder Caazetat · Hydrat bzw. bzw. Mg-acetat · Hydrat und 1,3-Dioxolan-2-on im Gewichtsverhältnis 1:10 oberflächennachvernetzt worden sind
oder vernetzte, zu 70 Mol% als Na-Salz vorliegende Polyacrylsäure, die auf native Wachsmaisstärke oder Polyvinylalkohol aufgepfropft ist und mit einer wäßrigen Lösung enthaltend $Al_2(SO_4)_3 \cdot$ 14 $H_2O$ und 1,3-Dioxolan-2-on im Gewichtsverhältnis 1:2 beschichtet und oberflächennachvernetzt worden sind,
ausgenommen sind,
**dadurch gekennzeichnet, dass** der Anteil des Polymerisates in bestimmten Bereichen der flüssigkeitsabsorbierenden Sauglage zwischen 60 und 100 Gew.-% liegt.

3. Hygieneartikel nach Anspruch 2, **dadurch gekennzeichnet, dass** die Komponente e) mit 0,1 bis 2,5 Gew.-%, bevorzugt mit 0,5 bis 1,5 Gew.-% und die Komponente f) mit 0,005 bis 0,5 Gew. %, bevorzugt mit 0,01 bis 0,2 Gew.-% eingesetzt worden ist.

4. Hygieneartikel nach den Ansprüchen 2 oder 3, **dadurch gekennzeichnet, dass** nur Wasser als Lösungsmittel für die Komponenten e) und f) eingesetzt wurde.

5. Hygieneartikel nach den Ansprüchen 2 bis 4, **dadurch gekennzeichnet, dass** die Komponenten e) und f) gemeinsam in einer wässrigen Lösung eingesetzt worden sind.

6. Hygieneartikel nach den Ansprüchen 1 bis 5, **dadurch gekennzeichnet, dass** die Gesamtmenge Wasser der getrennt oder gemeinsam zugegebenen wässrigen Lösungen 0 0,75 bis 5 Gew. %, bevorzugt 1 bis 4 Gew. %, bezogen auf das Polymerisat, betrug.

7. Hygieneartikel nach den Ansprüchen 1 bis 6, **dadurch gekennzeichnet, dass** als Komponente f) das Kation eines Alkali- oder ErdalkalimetallSalzes, eines Salzes von Zink, Eisen, Aluminium, Titan oder einem weiteren Übergangsmetall, oder von einem Doppelsalz zweier verschiedener Kationen oder von einer Mischung der Salze, bevorzugt von einem wasserlöslichen Aluminiumsalz ist.

8. Hygieneartikel nach den Ansprüchen 1 bis 7, **dadurch gekennzeichnet, dass** als Komponente e) Alkylencarbonate, vorzugsweise mit $C_4$ bis $C_{10}$, besonders bevorzugt mit $C_4$ bis $C_6$, im Ring eingesetzt worden sind.

9. Hygieneartikel nach Anspruch 7 und 8, **dadurch gekennzeichnet, dass** als Alkylencarbonat 1,3-Dioxolan-2-on und als Salz ein anorganisches Aluminiumsalz, vorzugsweise ein Aluminiumsulfat, eingesetzt worden ist.

10. Hygieneartikel nach den Ansprüchen 1 bis 9, **dadurch gekennzeichnet, dass** die Nachvernetzung bei Temperaturen von > 150°C bis 250°C, bevorzugt 160°C bis 220°C, besonders bevorzugt 170°C bis 200°C erfolgt ist.

**11.** Hygieneartikel nach den Ansprüchen 1 bis 10, **dadurch gekennzeichnet, dass** mindestens 50%, bevorzugt mindestens 75% der Säuregruppen der Monomereinheiten a) Carboxylgruppen sind.

**12.** Polymerisat nach den Ansprüchen 1 bis 11, **dadurch gekennzeichnet, dass** sich die Monomereinheiten a) von Acrylsäure und/oder Methacrylsäure ableiten.

**13.** Hygieneartikel nach den Ansprüchen 1 bis 12, **dadurch gekennzeichnet, dass** als Komponente d) Stärke und/ oder Polyvinylalkohol bzw. deren Derivaten eingesetzt worden sind.

**14.** Hygieneartikel nach den Ansprüchen 2 bis 13, **dadurch gekennzeichnet, dass** das Polymerisat bei einer Permeabilität (SFC) bis zu $70 \cdot 10^{-7}$s cm$^3$/g eine Retention (TB) von mindestens 27 g/g aufweist.

**15.** Hygieneartikel nach den Ansprüchen 2 bis 13, **dadurch gekennzeichnet, dass** das Polymerisat bei einer Permeabilität (SFC) von $> 70 \cdot 10^{-7}$ bis $150 \cdot 10^{-7}$s cm$^3$/g eine Retention (TB) von mindestens 25 g/g aufweist.

**16.** Hygieneartikel nach den Ansprüchen 2 bis 15, **dadurch gekennzeichnet, dass** das Polymerisat eine Flüssigkeitsaufnahme unter Druck (AAP 0,7) von mindestens 18 g/g aufweist.

## Claims

**1.** Use of pulverulent, polymers, which absorb water, aqueous or serous liquids as well as blood, which are cross-linked on the surface and constituted from

   a) 55-99,9 wt-% polymerized, ethylenically unsaturated, acid groups containing monomers, which are at least 25 mol% neutralized,
   b) 0-40 wt-% polymerized, ethylenically unsaturated monomers, which are copolymerizable with a),
   c) 0,1-5,0 wt-% of one or more cross-linking agents incorporated by polymerization,
   d) 0-30 wt-% of a water-soluble polymer,
   wherein the sum of the quantities by weight a) to d) is 100 wt-%, wherein the polymer is coated with and is post-cross-linked with heating with
   e) 0,01 to 5 wt-%, in relation to the polymer, of at least one organic surface post-cross-linking agent, with the exception of polyols, in the form of an aqueous solution and with
   f) 0,001-1,0 wt-%, in relation to the polymer, of a cation in the form of a salt dissolved in an aqueous solution,

wherein the total quantity of water of the coating solution is from 0,5 to 10 wt-%, in relation to the polymer and the ratio by weight of the salt to the post-cross-linking agent is within the range 1:0,8 to 1:4, and wherein cross-linked polyacrylic acids, which are up to 70 mol % present as Na-salts and which have been surface post-cross-linked with a solution containing Al$_2$(SO$_4$)$_3 \cdot$ 18 H$_2$O and 1,3-dioxolan-2-one in a ratio by weight of from 1:2 or 1:2,5 or 1:3,33 or 1:1,666 or 1:1,142 or 1:1 or with Al$_2$(SO$_4$)$_3 \cdot$ 14 H$_2$O and 1,3-dioxolan-2-one in a ratio by weight of 1:2 or with Al$_2$(SO$_4$)$_3 \cdot$ 18 H$_2$O and ethylene glycol diglycidyl ether in a ratio by weight of 1:1 or with aluminium chloride 6H$_2$O and 1,3-dioxolan-2-one in a ratio by weight of 1:1,43 or with iron(III) chloride $\cdot$ 6H$_2$O and 1,3-dioxolan-2-on in a ratio by weight of 1:1,43 or with Ca acetate $\cdot$ hydrate or Mg acetate $\cdot$hydrate and 1,3-dioxolan-2-one in a ratio by weight of 1:10 or cross-linked polyacrylic acid, which is 70 mol % present as a Na salt and which is grafted on native waxy maize starch or polyvinyl alcohol and has been coated and surface post-cross-linked with an aqueous solution containing Al$_2$(SO$_4$)$_3 \cdot$ 14 H$_2$O and 1,3-dioxolan-2-one in a ratio by weight of 1:2, are excepted, as absorption agent for water or aqueous liquids or blood in constructions for absorbing body liquids, **characterized in that** in specific regions of the structure the amount of the polymer is from 60 to 100 wt-%.

**2.** Hygienic articles, comprising an absorbent layer, which contains pulverulent polymers, post-cross-linked at the surface, and absorbing water, aqueous or serous liquids as well as blood, synthesized from

   a) 55-99,9 wt-% polymerized, ethylenically unsaturated, acid groups containing monomers, which are at least 25 mol% neutralized,
b) 0-40 wt-% polymerized, ethylenically unsaturated monomers, which are copolymerizable with a),
c) 0,1-5,0 wt-% of one or more cross-linking agents incorporated by polymerization,
d) 0-30 wt-% of a water-soluble polymer,
wherein the sum of the quantities by weight a) to d) is 100 wt-%, wherein the polymer is coated with and post-cross-linked with heating by
e) 0,01 to 5 wt-%, in relation to the polymer, of at least one organic surface post-cross-linking agent, with the exception of polyols, in the form of an aqueous solution and with
f) 0,001-1,0 wt-%, in relation to the polymer, of a cation in the form of a salt dissolved in an aqueous solution and

wherein the total quantity of water of the coating solution is from 0,5 to 10 wt-%, in relation to the polymer and the ratio by weight of the salt to the post-cross-linking agent is within the range 1:0,8 to 1:4,
and wherein
cross-linked polyacrylic acids, which are up to 70 mol % present as Na-salts and which have been surface post-cross-linked with a solution containing $Al_2(SO_4)_3 \cdot 18H_2O$ and 1,3-dioxolan-2-one in a ratio by weight of from 1:2 or 1:2,5 or 1:3,33 or 1:1,666 or 1:1,142 or 1:1 or with $Al_2(SO_4)_3 \cdot 14 H_2O$ and 1,3-dioxolan-2-one in a ratio by weight of 1:2 or with $Al_2(SO_4)_3 \cdot 18 H_2O$ and ethylene glycol diglycidyl ether in a ratio by weight of 1:1 or with aluminium chloride $\cdot 6H_2O$ and 1,3-dioxolan-2-one in a ratio by weight of 1:1,43 or with iron(III) chloride $\cdot 6H_2O$ and 1,3-dioxolan-2-on in a ratio by weight of 1:1,43 or Ca acetate $\cdot$hydrate or Mg acetate $\cdot$ hydrate and 1,3-dioxolan-2-one in a ratio by weight of 1:10
or cross-linked polyacrylic acid, which is 70 mol % present as a Na salt and which is grafted on native waxy maize starch or polyvinyl alcohol and has been coated and surface post-cross-linked with an aqueous solution containing $Al_2(SO_4)_3 \cdot 14H_2O$ and 1,3-dioxolan-2-one in a ratio by weight of 1:2,
are excepted,
**characterized in that** in specific regions of the liquid absorbent absorbing layer, the amount of the polymer is from 60 to 100 wt-%.

3. Hygienic articles according to claim 2, **characterized in that** the component e) has been utilized at from 0,1 to 2,5 wt-%, preferably at from 0,5 to 1,5 wt-% and the component f) has been utilized at from 0,005 to 0,5 wt-%, preferably at from 0,01 to 0,2 wt-%.

4. Hygienic articles according to claims 2 or 3, **characterized in that** only water has been utilized as a solvent for the components e) and f).

5. Hygienic articles according to claims 2 to 4, **characterized in that** the components e) and f) have been utilized together in an aqueous solution.

6. Hygienic articles according to claims 1 to 5, **characterized in that** the total quantity of water of the aqueous solutions, which are added separately or together was from 0,75 to 5 wt-%, preferably from 1 to 4 wt-%, in relation to the polymer.

7. Hygienic articles according to claims 1 to 6, **characterized in that** the component f) is a cation of an alkali metal or alkaline earth metal salt, a salt of zinc, iron, aluminium, titanium or of a further transition metal, or of a double salt of two different cations or of a mixture of the salts, preferably of a water-soluble aluminium salt.

8. Hygienic articles according to claims 1 to 7, **characterized in that** as component e) alkylene carbonates, preferably with $C_4$ to $C_{10}$, more preferably with $C_4$ to $C_6$ in the ring, have been used.

9. Hygienic articles according to claim 7 and 8, **characterized in that** 1,3-dioxolan-2-one has been used as alkylene carbonate and as salt an inorganic aluminium salt, preferably an aluminium salt.

10. Hygienic articles according to claims 1 to 9, **characterized in that** the post-cross-linking has taken place at temperatures of from > 150°C to 250°C, preferably at 160°C to 220°C, more preferably at 170°C to 200°C.

11. Hygienic articles according to claims 1 to 10, **characterized in that** at least 50 %, preferably at least 75 % of the acid groups of the monomer units a) are carboxyl groups.

12. Polymer according to claims 1 to 11, **characterized in that** the monomer units a) are derived from acrylic acid and/or methacrylic acid.

13. Hygienic articles according to claims 1 to 12, **characterized in that** as the component d) starch and/or polyvinyl alcohol or derivatives thereof have been utilized.

14. Hygienic articles according to claims 2 to 13, **characterized in that** the polymer has a retention (TB) of at least 27 g/g at a permeability (SFC) of up to $70 \cdot 10^{-7}$ cm$^3$sec/g.

15. Hygienic articles according to claims 2 to 13, **characterized in that** the polymer has a retention (TB) of at least 25 g/g at a permeability (SFC) of from $>70 \cdot 10^{-7}$ to $150 \cdot 10^{-7}$ cm$^3$sec/g

16. Hygienic articles according to claims 2 to 15, **characterized in that** the polymer has a liquid absorption at pressure (AAP 0,7) of at least 18 g/g.


**Revendications**

1. Utilisation de polymérisats en forme de poudre post-réticulés à la surface, absorbant de l'eau, des liquides aqueux ou séreux ainsi que du sang, constitués de

   a) 55-99,9 % en poids de monomères polymérisés, éthyléniquement insaturés et comprenant des groupes acides qui sont neutralisés à au moins 25 % en mole,
   b) 0-40 % en poids de monomères polymérisés, éthyléniquement insaturés, étant copolymérisables avec a),
   c) 0,1-5,0 % en poids d'un ou de plusieurs agents de réticulation incorporés par polymérisation,
   d) 0-30 % en poids d'un polymère soluble dans l'eau,
   le total des quantités en poids a) - d) comportant 100 % en poids,
   dans quel cas le polymérisat a été revêtu de
   e) 0,01 à 5 % en poids, par rapport au polymérisat, d'au moins un agent de post-réticulation de surface organique, à l'exception de polyols, en forme d'une solution aqueuse et de
   f) 0,001-1,0 % en poids par rapport au polymérisat, d'un cation en forme d'un sel dilué dans une solution aqueuse et post-réticulé sous réchauffement,

   la quantité totale d'eau de la solution de revêtement comportant 0,5 à 10 % en poids, par rapport au polymérisat et le rapport pondéral entre le sel et l'agent de post-réticulation se trouvant dans la plage de 1:0,8 à 1:4, et dans quel cas
   des acides polyacryliques réticulés qui se présentent comme sels de Na jusqu'à 70 % en mole et qui ont été post-réticulés à la surface avec une solution aqueuse comprenant du Al$_2$(SO$_4$)$_3 \cdot$ 18H$_2$O et du 1,3-dioxolanne-2-on en un rapport pondéral de 1:2, respectivement de 1:2,5, respectivement de 1:3,33, respectivement de 1:1,666, respectivement de 1:1,142, respectivement de 1:1 ou avec du Al$_2$(SO$_4$)$_3 \cdot$ 14 H$_2$O et du 1,3-dioxolanne-2-on en un rapport pondéral de 1:2 ou avec du Al$_2$(SO$_4$)$_3 \cdot$ 18 H$_2$O et de l'éther diglycilidique d'éthylèneglycol dans un rapport pondéral de 1:1 ou avec de l'alchlorure $\cdot$ 6H$_2$O et du 1,3-dioxolanne-2-on en un rapport pondéral de 1:1,43 ou avec de la chlorure de fer(III) $\cdot$ 6H$_2$O et du 1,3-dioxolanne-2-on dans un rapport pondéral de 1:1,43 ou avec de l'acétate de calcium $\cdot$ hydrate, respectivement de l'acétate de magnésium $\cdot$ hydrate et du 1,3-dioxolanne-2-on en un rapport pondéral de 1:10,
   ou de l'acide polyacrylique réticulé qui se présente à 70% en mole comme sel de Na et qui a été greffé sur de l'amidon cireux de maïs natif ou sur de l'alcool polyvinylique et qui a été revêtu d'une solution aqueuse contenant du Al$_2$(SO$_4$)$_3 \cdot$ 14 H$_2$O et du 1,3-dioxolanne-2-on dans un rapport pondéral de 1:2 et qui a été post-réticulé à la surface, sont exclus,
   comme agents d'absorption pour de l'eau ou pour des liquides aqueux ou du sang dans des constructions pour absorber des liquides corporels, **caractérisé en ce que** dans de certaines régions des constructions la quantité du polymérisat est entre 60 et 100 % en poids.

2. Articles d'hygiène, comprenant une couche absorbante absorbant des liquides, comprenant des polymérisats en forme de poudre, post-réticulés à la surface, absorbant de l'eau, des liquides aqueux ou séreux ainsi que du sang, constitués de

   a) 55-99,9 % en poids de monomères polymérisés, éthyléniquement insaturés et comprenant des groupes acides qui sont neutralisés à au moins 25 % en mole,
   b) 0-40 % en poids de monomères polymérisés, éthyléniquement insaturés étant copolymérisables avec a),
   c) 0,1-5,0 % en poids d'un ou plusieurs agents de réticulation incorporés par polymérisation,

d) 0-30 % en poids d'un polymère soluble dans l'eau,

le total des composants a) - d) comportant 100 % en poids,

dans quel cas le polymérisat est revêtu de

e) 0,01 à 5 % en poids, par rapport au polymérisat d'au moins un agent de post-réticulation de surface organique, à l'exception de polyols, en forme d'une solution aqueuse et de

f) 0,001-1,0 % en poids par rapport au polymérisat, d'un cation en forme d'un sel dilué dans une solution aqueuse et post-réticulé sous réchauffement,

la quantité totale d'eau de la solution de revêtement comportant 0,5 à 10 % en poids, par rapport au polymérisat et le rapport pondéral entre le sel et l'agent de post-réticulation étant dans la plage de 1:0,8 à 1:4, et dans quel cas des acides polyacryliques réticulés qui se présentent comme sels de Na jusqu'à 70 % en mole et qui ont été post-réticulés à la surface avec une solution aqueuse comprenant du $Al_2(SO_4)_3 \cdot 18\,H_2O$ et du 1,3-dioxolanne-2-on en un rapport pondéral de 1:2, respectivement de 1:2,5, respectivement de 1:3,33, respectivement de 1:1,666, respectivement de 1:1,142, respectivement de 1:1 ou avec du $Al_2(SO_4)_3 \cdot 14\,H_2O$ et du 1,3-dioxolanne-2-on en un rapport pondéral de 1:2 ou avec du $Al_2(SO_4)_3 \cdot 18\,H_2O$ et de l'éther diglycilidique d'éthylèneglycol dans un rapport pondéral de 1:1 ou avec de l'alchlorure $\cdot$ $6H_2O$ et du 1,3-dioxolanne-2-on en un rapport pondéral de 1:1,43 ou avec de la chlorure de fer(III) . $6H_2O$ et du 1,3-dioxolanne-2-on dans un rapport pondéral de 1:1,43 ou de l'acétate de calcium $\cdot$ hydrate, respectivement de l'acétate de magnésium $\cdot$ hydrate et du 1,3-dioxolanne-2-on en un rapport pondéral de 1 :10, ou de l'acide polyacrylique réticulé qui se présente à 70 % comme sel de Na et qui a été greffé sur de l'amidon cireux de maïs natif ou sur de l'alcool polyvinylique et qui a été revêtu d'une solution aqueuse contenant de $Al_2(SO_4)_3 \cdot 14\,H_2O$ et du 1,3-dioxolanne-2-on dans un rapport pondéral de 1:2 et qui a été post-réticulé à la surface, sont exclus, **caractérisés en ce que** dans de certaines régions de la couche absorbante absorbant des liquides la part du polymérisat est entre 60 et 100 % en poids.

3. Articles d'hygiène selon la revendication 2, **caractérisés en ce que** le composant e) est utilisé avec 0,1 à 2,5 % en poids, de préférence avec 0,5 à 1,5 % en poids et le composant f) avec 0,005 à 0,5 % en poids, de préférence avec 0,01 à 0,2 % en poids.

4. Articles d'hygiène selon les revendications 2 ou 3, **caractérisés en ce que** pour les composants e) et f) on utilise uniquement de l'eau en tant que solvant.

5. Articles d'hygiène selon les revendications 2 à 4, **caractérisés en ce que** les composants e) et f) sont utilisés communément dans une solution aqueuse.

6. Articles d'hygiène selon les revendications 1 à 5, **caractérisés en ce que** la quantité totale d'eau des solutions aqueuses ajoutées séparément ou communément comporte 0,75 à 5 % en poids, de préférence 1 à 4 % en poids, par rapport au polymérisat.

7. Articles d'hygiène selon les revendications 1 à 6, **caractérisés en ce que** comme composant f) est le cation d'un sel alcalin ou alcalinoterreux, d'un sel de zinc, de fer, d'aluminium, de titane ou d'un autre métal de transition, ou d'un sel double de deux cations différents ou d'un mélange des sels, de préférence d'un sel d'aluminium soluble à l'eau.

8. Articles d'hygiène selon les revendications 1 à 7, **caractérisés en ce que** comme composant e) on utilise des carbonates d'alcényle, de préférence avec $C_4$ à $C_{10}$, plus préférablement avec $C_4$ à $C_6$ dans le cycle.

9. Articles d'hygiène selon les revendications 7 et 8, **caractérisés en ce que** comme carbonate d'alcényle on utilise du 1,3-dioxolanne-2-on et comme sel un sel d'aluminium inorganique, de préférence un sulfate d'aluminium.

10. Articles d'hygiène selon les revendications 1 à 9, **caractérisés en ce que** la post-réticulation a lieu à des températures > 150°C à 250°C, de préférence 160°C à 220°C, plus préférablement à 170°C à 200°C.

11. Articles d'hygiène selon les revendications 1 à 10, **caractérisés en ce qu'**au moins 50 %, de préférence au moins 75 % des groupes acides des unités de monomères a) sont des groupes carboxyliques.

12. Polymérisat selon les revendications 1 à 11, **caractérisé en ce que** les unités de monomères a) sont dérivés d'acide acrylique et/ou d'acide méthacrylique.

13. Articles d'hygiène selon les revendications 1 à 12, **caractérisés en ce que** comme composant d) on utilise de l'amidon et/ou de l'alcool polyvinylique, respectivement leurs dérivés.

14. Articles d'hygiène selon les revendications 2 à 13, **caractérisés en ce qu'**à une perméabilité (SFC) jusqu'à 70 · $10^{-7}$s cm$^3$/g le polymérisat a une rétention (TB) d'au moins 27 g/g.

15. Articles d'hygiène selon les revendications 2 à 13, **caractérisés en ce qu'**à une perméabilité (SFC) de > 70 · $10^{-7}$ à 150 · $10^{-7}$s cm$^3$/g le polymérisat a une rétention (TB) d'au moins 25 g/g.

16. Articles d'hygiène selon les revendications 2 à 15, **caractérisés en ce que** le polymérisat a une absorption d'eau sous pression (AAP 0,7) d'au moins 18 g/g.